# EUROPEAN PATENT APPLICATION

(11) **EP 3 378 379 A1**
(43) Date of publication of application: **26.09.2018**
(21) Application number: 17162166.7
(22) Date of filing: 21.03.2017
(51) Int. Cl.: A61B 5/00

(54) **METHOD FOR CAPTURING THE THREE-DIMENSIONAL SURFACE GEOMETRY OF AN OBJECT**

(71) Applicant: a.tron3d GmbH, 9020 Klagenfurt am Wörthersee (AT)
(72) Inventor: JESENKO, Juergen, 9587 Riegersdorf (AT); WILHELMER, Thomas, 9991 Doelsach (AT)
(74) Representative: Beer & Partner Patentanwälte KG

(57) **Abstract**

At a method and device for capturing the three-dimensional surface geometry of an object (55), with at least one sensor (51) and at least one light source (52), the sensor (51) is a monochrome sensor (51) and the method comprises the following steps:
a. obtaining a first multidimensional information of the object (55) including a first pose, using the monochrome sensor (51),
b. illuminating the object (55) by the light source (52) in a first color,
c. taking a monochrome image of the illuminated object (55), using the monochrome sensor (51),
d. creating a second multidimensional information of the object (55) including a second pose, using the monochrome sensor (51),
e. combining the first and second pose of steps a. and d. to create an intermediate pose,
f. combining the monochrome image of step c. and the intermediate pose of step e. to create a colored model of at least a segment of the object (55).

## Description

The invention concerns a method and a device for capturing the three-dimensional surface geometry of an object, with at least one sensor and at least on light source.

In the state of the art it is known to capture intraoral structures with optical means, hence eliminating the need for uncomfortable physical impressions of a patient's dentition, as well as for storage of said impressions. While speed and precision have been permanently enhanced other aspects of the technology still lack development.

One of these aspects is producing colored scans of the intraoral structures. One step to provide such scans was to employ a multicolor sensor. However, multicolor sensors lack image sharpness and therefore reduce the required quality to provide precise three-dimensional information that may be used to construct dental products.

Hence it is an object of the invention to overcome the aforementioned obstacles and drawbacks while providing colored three-dimensional information.

This problem is solved by a method as described in claim 1 and a device as described in claim 8.

Monochrome sensors achieve a more detailed image with the drawback of no color information. In contrast, the color sensors have a lower image quality but color information. The invention connects the advantages of both systems. Still, for creating one color image, several, preferably three, monochrome images are advantageous. However, the number and specific color of the monochrome images can be chosen according to the depth and/or variety of color desired by those known in the art.

In a preferred embodiment the method comprises the following steps.

In a first step a first set of multidimensional information of the object is obtained. The method selected to create said information can be chosen by those known in the art and may contain but is not limited to stereography, pattern distortion, projection of stripes or the like. Independent of the method selected to create the multidimensional information it is required to know the position of the sensor relative to the object being scanned (first pose, step a.).

After multidimensional information and the first pose have been acquired the object is illuminated in a first color (step b.). Preferably the first color is part of a three-color set, such as "red-green-blue" (RGB) or "cyan-yellow-magenta" (CYM).

In the next step an image of the illuminated object is taken. This image provides color information for the one color the object has been illuminated with. If only simple color information is required, such as the difference between gum and teeth, the information of only one color may be sufficient. For this purpose colors in the blue and green range (450nm to 600nm) are preferred.

After the colored image has been taken a second multidimensional information including a second pose of the object is acquired.

Though in reality it might not be absolutely correct, for the purpose of the invention it is sufficiently precise to assume that the color image has been taken at a position right in the middle between the first and second pose. Therefore, an artificial pose is derived from the data of the first and second pose. This may be done for example via simple interpolation between both poses. The artificial pose and the color image are then combined to create at least partially colored 3D information of the segment of the object captured by the sensor(s) in the first and second pose.

A problem when employing separate monochrome images of the object is that these are usually taken from slightly different angles/positions. This can result in the so called Harris-shutter effect. This not only leads to incorrect color information but also may impair the general precision of the model. According to the invention this problem can be solved by "embedding" the color information within two sets of three-dimensional information. This way the color information is set in a correct context to the three-dimensional model. However, since the different colors are only partially overlapping distorted areas around the edges may occur. Despite those areas the method according the invention is preferable, for the Harris-shutter effect can be essentially avoided.

Of course only a portion of the surface of the virtual object can be colored this way, hence only a colored segment is created. However, with sufficient repetition of the process the whole virtual model can be colorized.

In case multicolor information is desired according to a preferred development of the invention these steps may be repeated with other colors (preferably from the same three color set as the first color image) and then combined to a multicolor three-dimensional information.

In a preferred but not limiting embodiment of the invention the three-dimensional surface geometry is noted within a truncated signed distance function (TSDF). The TSDF is advantageous over other notations as it can lead to higher precision and faster processing, as is describe in detail in US 2015/0024336 A1.

However, most TDSFs are created so that they may only bear a limited amount of information. A TSDF is noted within a three-dimension grid, the voxelgrid, each point within the grid (voxel) usually containing a fixed amount of data, for example 32 bit. Hence it can be possible that next to the information concerning the actual three-dimensional structure of the object scanned there is not enough "space" for further information such as color.

To solve this problem a second voxelgrid, the "color voxelgrid", can be provided, wherein all voxel-coordinates correspond to voxel-coordinates of the voxelgrid containing the TSDF. Instead of geometric/positional information color information can be stored in these voxels. Accordingly, if the color information to a voxel within the TSDF is required it can be read from the corresponding color voxelgrid at the same coordinates.

This embodiment has also a further advantage. The TSDF known from the state of the art has several properties and applicable operations that have been advantageously developed. Adding another level of additional information could slow down the processes known so far and hence impair the comfort for the user. Separating the information on the color from the other information, for example on geometry, number of updates, weight/precision or the like, enables the system to sustain the fast and easy operations known from the art.

To combine both voxelgrids an "inverse raycaster" is employed. Usually the raycaster obtains surface information including the geometrical structure as well as the color information to visualize the stored virtual model of the object. The employed "inverse raycaster" uses the monochrome image of the object and projects it onto the three-dimensional model using the artificial intermediate pose. The resulting information on which ray carrying which color "hits" the three-dimensional model at what coordinates is then used to create the color voxelgrid. A further explanation on employing a raycaster in general can be found in US 2015/0024336 A1.

Of course, with enough computational capacity an embodiment wherein the data is noted in only one voxelgrid may also be employed.

According to a preferred embodiment of the invention an amount of available three-dimensional information for a defined area of the object is counted and the steps a. to f. are executed for said area, when the amount exceeds a predefined value. Since the monochrome images of the illuminated object are usually not useful for creating depth information and hence information on the surface geometry, performing the steps a. to f. can slow down the process of obtaining the virtual three-dimensional model remarkably. First collecting a sufficient amount of information on a defined area of the object before taking the first illuminated monochrome image helps to overcome this drawback. The necessity of realizing this embodiment of course is dependent on the desired quality of three-dimensional and color information and the performance of the computational device processing the data, respectively.

A defined area of the object may for example be all parts of the object within a certain portion of the TSDF. For example, the voxelgrid may be divided in blocks of 8³ voxel.

In a further advanced embodiment of the invention the monochrome images of the illuminated object are used to produce additional three-dimensional information of the surface of the object. For this purpose the so called "shape by shading" method may be employed. For this method the lighter and darker areas of the image are analyzed and depth information is derived. This is based on the effect that surfaces essentially normal to the viewing direction of the sensor reflect more light than surfaces not normal to the viewing direction of the sensor. The darker a portion of the monochrome image is perceived the stronger it is "bent away" from the sensor. These changes in lighting can be used to derive information on the structure of the surface.

Further preferred and advantageous embodiments of the invention are object of the dependent claims.

In the following a depiction of a preferred embodiment of the invention is described. The described embodiments are solely meant to exemplify the invention, which is not limited to the shown examples but may be implemented in a wide range of embodiments.

### Brief description of the drawings:

- Fig. 1: shows an exemplary embodiment of a method according to the invention,
- Fig. 2: shows an exemplary for combining color and three-dimensional information,
- Fig. 3: illustrates an "inverse raycaster",
- Fig. 4: shows schematically the color information from three monochrome images fitted to a three-dimensional model of the object,
- Fig. 5: shows a schematic depiction of an exemplary embodiment of a device according to the invention.

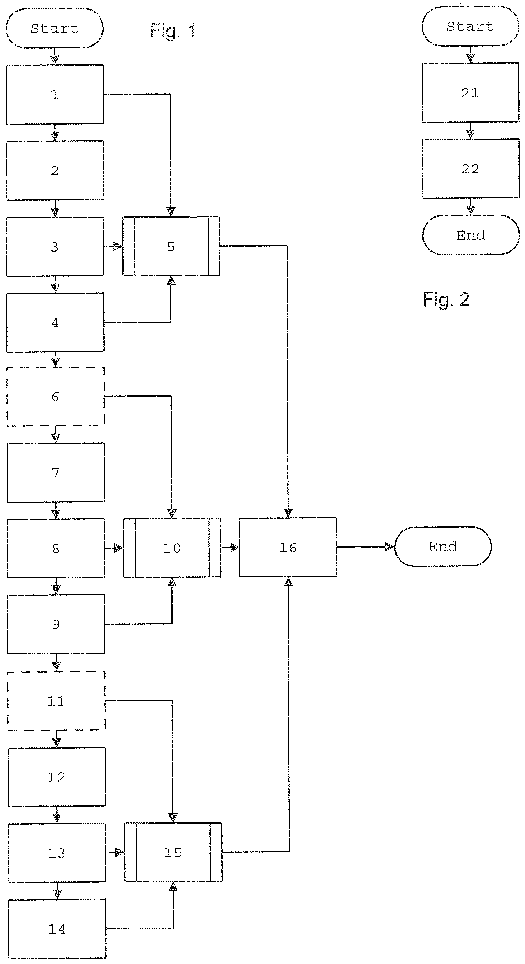

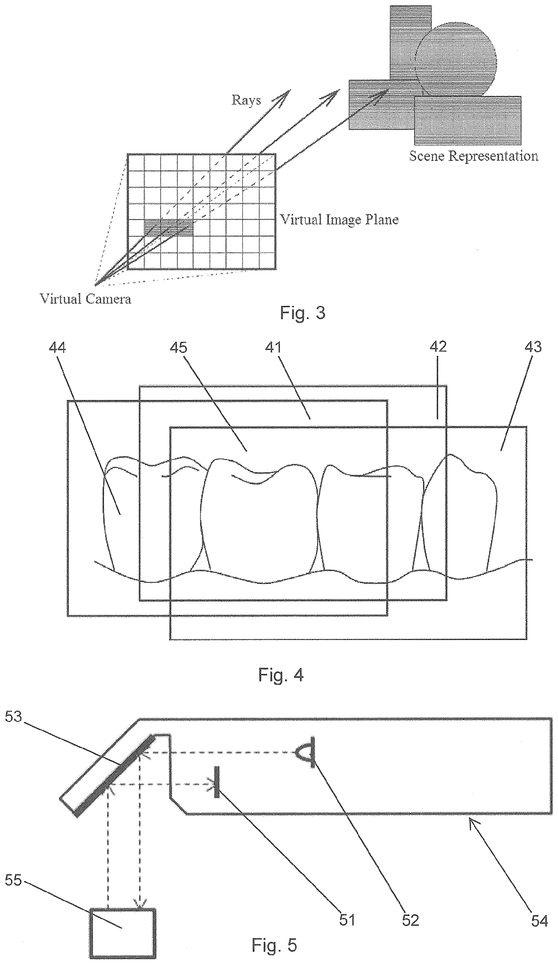

In step 1 of Fig. 1 a first set of three-dimensional information is obtained and saved. Said information contains the position and orientation of an optical sensor 51 (see fig. 5) of the scanner relative to the object 55 (see fig. 5) scanned (pose).

In step 2 the object 55 is illuminated in a first color. For example the object 55 may be illuminated in red color, but other colors are possible and may be chosen at the discretion of a skilled person.

In step 3 a monochrome image of the object 55 illuminated in the selected color is taken. Next to visible information also the color used to illuminate the object 55 is saved for further use.

In step 4 further three-dimensional information including a second pose is obtained. The three-dimensional information, the monochrome picture and the further three-dimensional information being obtained in immediate succession.

In step 5 two subsequent steps (as shown in Fig. 2) create an artificial intermediate pose of the first and second pose and combine it with the monochrome image, thereby creating a first colored subset of the virtual model.

The steps 6 to 10 basically repeat the steps 1 to 5 but with another color, if multicolor information is desired. Since step 4 already provides three-dimensional information including a pose, it can be used instead of step 6, rendering it optional. However, it is also possible that additional three-dimensional information is obtained in-between steps 4 and 6, for example to provide a denser and/or more precise virtual model of the object 55.

Accordingly steps 11 to 15 again repeat the steps 1 to 5 with yet another color. Though the method may be carried out with less colors a most natural colorization can be obtained by employing either red, green and blue or magenta, yellow and cyan.

If it is desired, for example to fasten the process, to use fewer colors to create the monochrome images a so called CLUT (color look-up table) may be employed. A CLUT uses the fact that certain materials among the intraoral structures usually have a similar color with a similar composition of the possible projected colors. For example gum tissue has very little range in the amount of green or blue that can be measured, when illuminated accordingly. So it can be identified even when only illuminated in one of these colors. Based on this information a differentiation between "gum" and "rest" (usually teeth) can be made. Typical coloring can then be selected from the table without precise knowledge of the real color and can be used to create a colorization of the image that still can be perceived as natural.

If two or three different colors are used to illuminate the monochrome images the two or three colored subsets from steps 5, 10 and 15 may then be combined in step 16.

Fig. 2 shows the process of step 5 in more detail. First in step 21 the intermediate pose between the poses of step 1 and step 4 is created. Then in step 22 the virtual object, as it has been obtained so far, is "observed" from the point of view of the intermediate pose by means of a virtual camera. Further explanations and uses of the employment of a virtual camera can be found in European patent application EP 2 886 043. Along the viewing rays of the virtual camera the monochrome image is positioned like a virtual transparency (see also Fig. 3) and projected onto the virtual object. Hence instead of obtaining color and surface information like a ray caster would do, it assigns color information to the virtual object and can be regarded a "inverse raycaster".

Fig. 4 illustrates three different "color-patches" 41, 42, 43 assigned to the virtual model 44. In the middle portion 45 where all three "color-patches" overlap the final colorization is achieved.

Fig. 5 shows a strongly simplified depiction of a device with a sensor 51, a light source 52 and a mirror 53 arranged within a handpiece 54. Light (or illumination, symbolically depicted as arrows) is emitted by the light source 52 and hits the mirror 53. According to a preferred embodiment the mirror 53 only reflects light emitted by the light source 52. This helps to minimize possible errors and distortions caused by any ambient lighting. The mirror 53 then reflects the light onto an object 55. The object 55 reflects the light back onto the mirror 53.

Due to the nature of intraoral structures it can happen that the light that hits the object 55 is slightly changed. For example it is known that ultra violet light, when shone onto teeth, enters these and is slightly shifted towards a greener" color before it is reflected back.

In an advanced embodiment of the invention therefore the mirror 53 also or exclusively reflects light reflected by the object 55 that has been emitted by the light source 52. If the mirror 53 exclusively reflects light reflected by the object 55 that has been emitted by the light source 52, it is advantageous to shine the light from the light source 52 directly on the object 55.

After being reflected from the mirror 53, the light enters the sensor 51. To further avoid errors and/or distortions of ambient lighting the sensor 51 may also comprise a filter that only can be passed by either light produced by the light source 52 and/or reflected by the object 55 after being illuminated by the light source 52.

A combination of only partially reflective mirrors 53 and sensor filters is of course conceivable and may be chosen advantageously by a skilled person.

## Claims

1. Method for capturing the three-dimensional surface geometry of an object (55), with at least one sensor (51) and at least one light source (52), **characterized in that** the sensor (51) is a monochrome sensor (51) and that the method comprises the following steps:
a. obtaining a first multidimensional information of the object (55) including a first pose, using the monochrome sensor (51),
b. illuminating the object (55) by the light source (52) in a first color,
c. taking a monochrome image of the illuminated object (55), using the monochrome sensor (51),
d. creating a second multidimensional information of the object (55) including a second pose, using the monochrome sensor (51),
e. combining the first and second pose of steps a. and d. to create an intermediate pose,
f. combining the monochrome image of step c. and the intermediate pose of step e. to create a colored model of at least a segment of the object (55).

2. Method according to claim 1, **characterized in that** the method comprises the step
g. repeating the steps a. to f. whereby the object (55) is illuminated by the light source (52) with at least a second color during step b.

3. Method according to claim 2, **characterized in that** the first and/or second color is part of a three color set, preferably "red-green-blue" (RGB) or "cyan-yellow-magenta" (CYM).

4. Method according to one of the claims 1 to 3, **characterized in that** the three-dimensional surface geometry is noted within a truncated signed distance function (TSDF).

5. Method according to claim 4, **characterized in that** the TSDF is noted within a first voxelgrid, that a second voxelgrid corresponding to the first voxelgrid is provided and that color information is noted in the second voxelgrid.

6. Method according to one of the claims 1 to 5, **characterized in that** an amount of available three-dimensional information for a defined area of the object (55) is counted and that the steps a. to f. are executed for said area, when the amount exceeds a predefined value.

7. Method according to one of the claims 1 to 6, **characterized in that** the monochrome images of the illuminated object (55) are used to produce additional three-dimensional information of the surface of the object (55).

8. Device for capturing the three-dimensional surface geometry of an object (55), with at least one sensor (51), at least on light source (52) and a computational device, **characterized in that**, the sensor (51) is a monochrome optical sensor (51) and the light source (52) is a multicolor light source (52).

9. Device according to claim 8, **characterized in that** the light source (52) is a RGB or CYM light source.

10. Device according to one of the claims 8 or 9, **characterized in that** the device comprises a filter and **in that** only light in a wavelength produced by the at least one light source (52) can pass the filter.

11. Device according to one of the claims 8 or 9, **characterized in that** the device comprises a filter and **in that** only light in a wavelength produced by the at least one light source (52) and reflected by an intraoral structure can pass the filter.

12. Device according to one of the claims 10 or 11, **characterized in that** the filter is a coating on the sensor (51).

13. Device according to one of the claims 8 to 12, **characterized in that** the device comprises a mirror (53) and **in that** only light in a wavelength produced by the at least one light source (52) is reflected.

14. Device according to one of the claims 8 or 12, **characterized in that** the device comprises a mirror (53) and **in that** only light in a wavelength produced by the at least one light source (52) and reflected by an intraoral structure is reflected by the mirror (53) .
